# EUROPEAN PATENT APPLICATION

(11) **EP 3 231 435 A1**
(43) Date of publication of application: **18.10.2017**
(21) Application number: 17164469.3
(22) Date of filing: 15.07.2010
(51) Int. Cl.: A61K 35/20, A61K 38/44, A61K 38/06, A61K 36/815, A61K 31/593, A61K 31/375, A61K 31/202, A61K 31/198, A61K 31/195, A61K 9/107, A61P 21/00, A61P 21/06, A61K 45/06, A23L 33/10, A23L 33/15, A23L 33/16, A23L 33/19

(54) **METHODS OF ATTENUATING THE LOSS OF FUNCTIONAL STATUS**

(30) Priority: 20.07.2009 US 226806 P; 13.05.2010 US 334247 P
(62) Divisional of application: 10747335.7
(71) Applicant: Nestec S.A., 1800 Vevey (CH)
(72) Inventor: MILLER, Kevin, Plymouth, MN 55447 (US); JURK, Ingo, 1196 GLAND (CH); ROUGHEAD, Zamzam, Kabiry (Fariba), Plymouth, MN Minnesota 55446 (US)
(74) Representative: Chautard, Cécile

(57) **Abstract**

Nutritional compositions and methods of using the nutritional compositions with exercise to attenuate the loss of functional status are provided. In a general embodiment, the present disclosure provides a nutritional composition including whey protein, and Vitamin D. The nutritional composition and exercise can be specifically used to attenuate the loss of functional status, especially in the elderly.

## Description

### CROSS REFERENCE TO RELATED APPLICATIONS

The instant application claims the benefit of prior U.S. Provisional Application Serial No. 61/226,806 filed on 20 July 2009 and prior U.S. Provisional Application Serial No. 61/334,247 filed on 13 May 2010.

### DETAILED DESCRIPTION

As used herein, "about," is preferably understood to refer to numbers in a range of numerals. Moreover, all numerical ranges herein should be understood to include all integer, whole or fractions, within the range.

As used herein the term "amino acid" is preferably understood to include one or more amino acids. The amino acid can be Alanine, Arginine, Asparagine, Aspartate, Citrulline, Cysteine, Glutamate, Glutamine, Glycine, Histidine, Hydroxyproline, Hydroxyserine, Hydroxytyrosine, Hydroxylysine, Isoleucine, Leucine, Lysine, Methionine, Phenylalanine, Proline, Serine, Taurine, Threonine, Tryptophan, Tyrosine, and Valine or a combination thereof, and maybe included in an embodiment of the invention.

As used herein the term "antioxidant" is preferably understood to include any one or more of various substances (as beta-carotene (a vitamin A precursor), vitamin C, vitamin E, and selenium) that inhibit oxidation or reactions promoted by Reactive Oxygen Species (ROS) and other radical and non-radical species. Additionally, antioxidants are molecules capable of slowing or preventing the oxidation of other molecules. As used herein, non-limiting examples of antioxidants include carotenoids, coenzyme Q10 ("CoQ10"), flavonoids, glutathione Goji (Wolfberry), hesperidine, Lactowolfberry, lignan, lutein, lycopene, polyphenols, selenium, vitamin A, vitamin B1, vitamin B6, vitamin B12, vitamin C, vitamin D, vitamin E, and combinations thereof, and maybe included in an embodiment of the invention.

As used herein, "effective amount" is preferably an amount that prevents a deficiency, treats a disease or medical condition in an individual or, more generally, reduces symptoms, manages progression of the diseases or provides a nutritional, physiological, or medical benefit to the individual. A treatment can be patient- or doctor-related. In addition, while the terms "individual" and "patient" are often used herein to refer to a human, the invention is not so limited. Accordingly, the terms "individual" and "patient" refer to any animal, mammal or human having or at risk for a medical condition that can benefit from the treatment.

As used herein, mammal includes but is not limited to rodents, aquatic mammals, domestic animals such as dogs and cats, farm animals such as sheep, pigs, cows and horses, and humans. Wherein the term mammal is used, it is contemplated that it also applies to other animals that are capable of the effect exhibited or intended to be exhibited by the mammal.

As used herein, animals include, but is not limited to mammals which includes but is not limited to rodents, aquatic mammals, domestic animals such as dogs and cats, farm animals such as sheep, pigs, cows and horses, and humans. Wherein the terms animal or mammal or their plurals are used, it is contemplated that it also applies to any animals that are capable of the effect exhibited or intended to be exhibited by the context of the passage.

As used herein, "elderly" is preferably a human that is sixty-five years of age or older, more preferably 75 years or age or older.

As used herein the term "patient" is preferably understood to include an animal, especially a mammal, and more especially a human that is receiving or intended to receive treatment, as it is herein defined.

As used herein, "Short term administrations" are preferably continuous administrations for less than 6 weeks.

As used herein, "Long term administrations" are preferably continuous administrations for more than 6 weeks.

As used herein, "complete nutrition" are preferably nutritional products that contain sufficient levels of macronutrients (protein, fats and carbohydrates) and micronutrients to be sufficient to be a sole source of nutrition for the animal to which it is being administered to.

As used herein, "incomplete nutrition" are preferably nutritional products that do not contain sufficient levels of macronutrients (protein, fats and carbohydrates) or micronutrients to be sufficient to be a sole source of nutrition for the animal to which it is being administered to.

As used herein, a "tube feed" is preferably a complete or incomplete nutritional products that are administered to an animal's gastrointestinal system, other than through oral administration, including but not limited to a nasogastric tube, oral gastric tube, port, such as a chest wall port that provides access to the stomach, jejunum and other suitable access ports, and/or Percutaneous Endoscopic Gastrostomy (PEG).

As used herein the term "minerals" is preferably understood to include boron, calcium, chromium, copper, iodine, iron, magnesium, manganese, molybdenum, nickel, phosphorus, potassium, selenium, silicon, tin, vanadium, zinc, and combinations thereof, and maybe included in an embodiment of the invention.

As used herein the term "vitamin" is preferably understood to include any of various fat-soluble or water-soluble organic substances (non-limiting examples include vitamin A , Vitamin B1 (thiamine), Vitamin B2 (riboflavin), Vitamin B3 (niacin or niacinamide), Vitamin B5 (pantothenic acid), Vitamin B6 (pyridoxine, pyridoxal, or pyridoxamine, or pyridoxine hydrochloride), Vitamin B7 (biotin), Vitamin B9 (folic acid), and Vitamin B12 (various cobalamins; commonly cyanocobalamin in vitamin supplements), vitamin C, vitamin D, vitamin E, vitamin K, folic acid and biotin) essential in minute amounts for normal growth and activity of the body and obtained naturally from plant and animal foods or synthetically made, pro-vitamins, derivatives, analogs, and maybe included in an embodiment of the invention.

As used herein, the terms "treatment" and "treat" is preferably to both prophylactic or preventive treatment and curative or disease-modifying treatment, including treatment of patients at risk of contracting a disease or suspected to have contracted a disease, as well as patients who are ill or have been diagnosed as suffering from a disease or medical condition. The terms "treatment" and "treat" also refer to the maintenance and/or promotion of health in an individual not suffering from a disease but who may be susceptible to the development of an unhealthy condition, such as nitrogen imbalance or muscle loss.

Nutritional products is preferably understood to further include any number of optional additional ingredients, including conventional food additives, for example one or more, acidulants, additional thickeners, buffers or agents for pH adjustment, chelating agents, colorants, emulsifies, excipient, flavor agent, mineral, osmotic agents, a pharmaceutically acceptable carrier, preservatives, stabilizers, sugar, sweeteners, texturizers, and/or vitamin. The optional ingredients can be added in any suitable amount.

As used herein, "whey protein" is intended to include whey protein micelles, whey protein concentrate, whey protein hydrolysates, and whey protein isolate and combinations thereof.

Muscle loss There are two factors that contribute to the loss of muscle mass and also the loss of muscle function. However, it is important to note that muscle mass and function are not necessarily correlated.
- The first factor is a depression in protein synthesis. Decreases in protein synthesis can occur in people with sub-optimal nutrition, but also in sedentary people with adequate dietary intake. Lower levels of protein synthesis are also a natural process of ageing.
- The second factor contributing to loss of muscle mass (and muscle function) is accelerated catabolism. Both disease and injury insult to the body or tissues are mechanisms that increase the degradation of muscle. Inflammation caused by injury, sickness, or even ageing is also included as initiators of muscle breakdown.

Skeletal muscle is in a constant state of flux with approximately equal degrees of muscle building and muscle breakdown. If either of these two conditions is altered, the total amount of muscle mass is affected.

Individuals who have been in an accident, suffered a stroke, general malaise, frail elderly, etc may be confined to their beds. The loss of contractile activity by the muscle reduces the body's signals for protein synthesis. Because the remodeling process in muscle occurs continuously, the loss of protein synthesis with constant muscle breakdown tips the balance towards net muscle loss. However, there are interventions to slow the rate of loss. The three mechanisms are physical activity, nutrition, and pharmaceuticals.

In the elderly, as well as recovering patients, the act of walking and other weight bearing activities are sufficient for release of myoD protein to activate satellite cells for muscles to repair and rebuild. Increased activity beyond walking results in greater benefits. In addition to muscle, activity stimulates the generation of more mitochondria within the myocytes, which are necessary for energy production to maintain muscular function. Use of muscle is considered to be the best method available to maintain and grow muscle and function. Disuse results in atrophy.

On the other hand, disease and infections can increase the rate of catabolism which breaks down muscle. In this case, the balance shifts in favor of muscle loss despite normal protein synthesis. The muscle breakdown may be the result of elevated inflammatory cytokines or the use of muscle proteins as a source of energy. Protein is a readily available source of energy and malnutrition can also cause these stores to be mobilized quickly, unlike fat tissue which takes much longer.

The correlation between loss of muscle mass or muscle function and morbidity is well known. The patient prognosis, likelihood of acquiring a nosocomial infection, and length of stay in hospital are all correlated to the patient's muscle mass. A decrease in muscle mass and function can not only reduce physical activity, but have metabolic effects including decreased bone density, obesity, and impaired glucose tolerance. It has been shown that a loss of approximately 3-5 % of muscle mass per decade occurs after the age of 30 years, although this decline is higher after the age of 60 years and older.

Systemic inflammation, including subclinical inflammation, in the elderly is part of the etiology of sarcopenia. It was recently reported that elevated CRP (inflammatory marker) and low hemoglobin were associated with decreased muscle strength. Interleukin-6 and CRP, but not TNF-alpha, are reported elsewhere to correlate to physical function. These sub-clinical levels of inflammation often go unreported and continue to cause decline in the patient's functionality until significant muscle is lost.

Pharmacological agents, such as anabolic steroid hormones and growth hormone, are occasionally prescribed as the endogenous levels of growth hormone and androgens decrease with age. However, these agents may also put the patient at risk for development of serious complications that include initiation of cancer. Nutritional interventions are believed to provide an efficacious treatment program without the risks associated with drug therapy.

Physical interventions such as walking, stair climbing, rising from a chair, and load carrying are functional activities that deteriorate with muscle loss. The loss of type II muscle fibers, results in diminished strength and power-generating capacity. There is a significant correlation between strength and leg power and maximal walking speed and stair-climbing height. Rantanen and Avela have described critical ranges of leg power for different walking speeds, suggesting that below the critical range the probability of walking at a particular speed is low. Interventions have been investigated that include pharmaceuticals, aerobic exercise, and specific compounds.

Selection of an appropriate physical intervention is critical to success. Endurance training does not appear effective in preserving muscle mass and ameliorating the progression of sarcopenia.

Benefits of engaging in regular resistance training include an increase in basal metabolism and limb perfusion, bone mineral density, as well as improved insulin sensitivity, and lipid and lipoprotein profiles. Although the list of benefits induced by resistance training is impressive, the magnitude of these health benefits may be substantially smaller than those achieved by endurance training, and there may be unfavorable effects associated with resistance training as well.

Recent studies reported that individuals habitually performing strength training exhibit a greater rate of age-related arterial stiffening and that a period of strenuous resistance training increases arterial stiffness. Because arterial stiffening precedes and may even initiate the development of elevated blood pressure, resulting in the hypertension that often leads to a major clinical event, these findings are alarming. Fortunately, endurance training concurrently performed with resistance training appears to negate the arterial stiffening effects of resistance training. This exercise regimen is consistent with the current physical activity recommendation to perform both endurance and resistance training on a daily basis. Other comparative studies demonstrated that the combination of resistance and endurance training results in better cardiovascular adaptations than endurance training alone, especially in the older cardiac patient population.

However, it is known that heavy exercise causes increased calcium loss through sweat, and the body does not compensate for this by reducing calcium loss in the urine. The result can be a net calcium loss great enough so that it presents health concerns for menopausal women. One study found that use of an inexpensive calcium supplement (calcium carbonate), taken at a dose of 400 mg twice daily, is sufficient to offset this loss.

Oxidative stress is increased during exercise and the damage potential to elderly tissues is likely to increase cellular damage that signals the proteolytic remodeling to correct damage.

Nutrition as a solution to issues in therapeutic exercise: There are specific nutrients that are reported to increase protein synthesis and have been used, with limited success, to promote the retention or building of lean muscle mass.

The most notable include: branched chain amino acids (BCAAs) (valine, leucine, and isoleucine). BCAAs may be administered in their free forms, as dipeptides, as tripeptides, as polypeptides, as BCAA-rich protein, and/or as protein manipulated to enrich the BCAA content. Dipeptides, tripeptides and polypeptides may include two or more BCAAs. Where non-BCAAs are included in a dipeptide, tripeptide, or polypeptide preferred amino acids include alanine and glycine, but non-BCAAs may be any of the dispensable or indispensable (essential or non-essential) amino acids. For example, preferred dipeptides include, but are not limited to, alanyl-leucine, alanyl-isoleucine, alanyl-valine, glycyl-leucine, glycyl-isoleucine, and glycyl-valine. Leucine precursors, such as pyruvate, and metabolites, such as α-hydroxuisocaproate,□ □-hydroxy-□-methylbutyrate and α□ketoisocaproate, whey protein (includes high leucine whey protein); creatine; antioxidants including bioactives such as lycopene, pycnogenol; quercetin. Antioxidants that may influence mitochondria include: genistein (soy), epigallocatechin (green tea), lipoic acid (ALA), X-tocopherol, dihydroascorbic acid (Vit C), and ubiquinone (Coenzyme Q10).

Age-related alterations in amino acid metabolism that are linked to muscle loss may be overcome by 1) supplementing excess leucine into the diet, 2) increasing protein intake or 3) exercise, which improve activation of translation initiation and muscle protein synthesis. Leucine has poor organoleptic properties and therefore is difficult to supplement orally therefore, dipeptides, polypeptides, leucine precursors, leucine metabolites and proteins high in leucine are the preferred method of administration.

Somatopause is the process of reduced growth hormone and IGF-1 as we age. This reduction in important anabolic hormones leads to reduced lean body mass (sarcopenia) and bone mineral density over time (osteopenia, osteoporosis). Certain dietary factors such as high protein diets as well as and dietary Zn and Cu have been shown to help increase serum IGF. Furthermore, the composition provides other important nutrients such as high levels of vitamin D which synergistically increases the efficacy of other anabolic hormones such as insulin (note that as we age, we also become insulin resistant). Therefore, the unique combination of these key nutrients will synergistically create a favorable physiological response leading to improved overall musculoskeletal health. The combination of such formulation with resistance exercise will also lead to synergistic benefits not realized with an equivalent amount of essential amino acids, or other key ingredients alone.

Vitamin D in skeletal muscle activates the signaling molecule protein kinase C which results in subsequent calcium release, increasing the calcium pool, which is essential for skeletal muscle contraction. Furthermore, animal data indicates that exogenous 25-OH increases skeletal muscle protein synthesis. Administration of vitamin D in deficient rats resulted in increased muscle mass, weight gain, and a decreased rate of myofibrillar protein degradation. These findings are corroborated by human biopsy studies that compared muscle biopsies of vitamin D deficient patients, pre- and post-vitamin D supplementation. The biopsies demonstrated atrophy of type II muscle fibers pre-supplementation while significant improvements were observed post-supplementation (type II muscle fibers are a type of skeletal muscle used for short bursts of power and speed).

Clinical studies have suggested that vitamin D insufficiency is associated with poor lower extremity performance. Several randomized, controlled intervention trials have found that vitamin D supplementation in amounts that bring the treated group's mean serum 25-OH Vitamin D level to 66-84 nmol/L improves lower extremity muscle performance in the elderly. Additionally, serum 25-OH Vitamin D was the common contributor to physical fitness indices (androidal fat mass, lean mass, balance, handgrip strength) in healthy postmenopausal women. The proposed nutritional formula will seek to significantly augment the circulating levels of serum 25-OH Vitamin D in the elderly utilizing a concentrated, low volume approach.

Exercise increases the energy need by the cell. Oxidative phosphorylation to create ATP results in the generation of free radicals (e.g., superoxide) that can damage the cells. Athletes that generate high amounts of these oxidative radicals typically have both good nutrition and a good endogenous antioxidant levels (Superoxide dismutases, glutathione, etc.) necessary to control exercise-induced damage. The elderly and sick do not possess the ability to efficiently defend against these free radicals. Glutathione (Gln-Cys-Gly), is an intracellular antioxidant produced by the body from three amino acids. Limiting the supply of glutamine, Cysteine (cystine) or glycine will reduce glutathione synthesis and levels. The use of whey protein is one source of Cysteine (cystine).

Physical interventions that include strategies to promote protein synthesis in a nutritionally compromised patient will result in potentially negative results. Protein synthesis increases the demand for amino acids to create new proteins. If dietary protein intake is inadequate to meet demands, the muscles and other protein-rich tissues are degraded to liberate amino acids. The elderly, patients recovering from an injury, etc are at the greatest risk as their nutritional intakes, particularly protein, are well documented to be suboptimal.

Exercise induced oxidative phosphorylation increases the consumption of oxygen by the cell (e.g., contracting muscle cells). When oxygen supply is inadequate to support this method of energy production, the body switches to anaerobic metabolism. The result of which is lactate production. The aged, recovering patient etc. do not reach a level of performance that limits oxygen supply like athletes, but poor circulation, low hemoglobin levels, chronic conditions including COPD all limit 02 carrying capacity. As a result, lactate production lowers the muscle pH causing sub-acute to clinical metabolic acidosis. Beta-alanine, a metabolic buffer within the cells, can be supplemented into the diet of these individuals to reduce the stress on the muscle..

The prior art teaches that dietary interventions, including elevated protein, have not been entirely successful in improving physical function. Nutrition is a stimulant of protein synthesis, but inadequate to make a clinically relevant difference. The combination of specific exercise program and specific nutritional intervention suggests the benefits of each treatment modality may be synergistic.

Although the protein requirements for elderly individuals was recently confirmed by the Institute of Medicine to be similar to adults 55 years and younger (0•8 g/kg/d; Food and Nutrition Board and Institute of Medicine, 2002), Campbell et al. (1994) found that elderly subjects fed an iso-energetic diet with the recommended protein (0•8 g/kg/d) for 2 weeks reduced N excretion and mid-thigh muscle area. The results suggest the recommendation is not adequate to meet the metabolic requirements of healthy elderly. Nutritional interventions for the prevention and treatment of sarcopenia are exciting as a result of easy applicability and safety, but attempts to improve muscle mass in elderly subjects with protein supplementation have been largely unsuccessful.

Proper nutrition is vital to assist bone repair and prevent further falls, particularly in malnourished patients. Vitamin D, calcium and protein supplementation is associated with an increase in hip Bone Mineral Density and reduction in falls. Rehabilitation is essential to improve functional disabilities and survival rates. Fall prevention and functional recovery strategies should include patient education and training to improve balance and increase muscle strength and mobility.

An intervention for sarcopenia and physical frailty that includes nutrition can also benefit from the inclusion of vitamins and minerals, including zinc, magnesium, folate, vitamins C and B12. The carotenoids are of particular interest in the nutritional interventions described for improvement of physical function.

Timing of nutritional supplementation also plays a role in the efficacy that can be realized from the combination of exercise and nutrition. The use of nutrition as a stimulant to protein synthesis and recovery has been described for proteins and energy, but not for vitamins, minerals, and bioactive components. In order for the antioxidant components to minimize damage they must be administered early enough to be bioactive in the individual during and after exercise. Therefore, a supplement delivers the greatest benefit between 30 minutes and 1 hour prior to exercise and less than 1 hour to 30 minutes following. Ideally, nutritional interventions are delivered between 30 minutes before or after exercise.

Pulse feeding of the nutritional supplement several times per day in conjunction with meals and/or following exercise is a preferred method of delivery. Delivery of a concentrated or higher level of protein typically consumed at a meal will raise the blood amino acids levels (i.e. branched chain amino acids) and allow an anabolic threshold to be reached in which to increase fractional rates of protein synthesis in skeletal muscle. The stimulation of protein synthesis must be sufficient such that in the context of protein turnover (i.e. synthesis-breakdown) the net anabolic response is positive and eventually leads to lean mass accretion over time. Additionally, ingestion of the nutritional product following exercise will further augment the anabolic response given the increased blood flow and enhanced perfusion of nutrients (i.e. amino acids) to the skeletal muscle in elderly individuals.

Exercise recommend from the American College Sports Medicine: A combination of endurance (aerobic) and resistance exercise may provide additional benefits to the individual that are not fully realized with compliance to one or the other alone.

### The ACSM/AHA Physical Activity Recommendations:

### Endurance exercise for older adults:

- Frequency: Moderate-intensity activities, accumulate at least 30 or up to 60 (for greater benefit) min/d in bouts of at least 10 min each to total 150-300 min/wk, at least 20-30 min/d or more of vigorous-intensity activities to total 75-150 min/wk, an equivalent combination of moderate and vigorous activity.
- Intensity: On a scale of 0-10 for level of physical exertion, 5-6 for moderate-intensity and 7 to 8 for vigorous intensity.
- Duration: For moderate-intensity activities, accumulate at least 30 min/d in bouts of at least 10 min each or at least 20 min/d of continuous activity for vigorous-intensity activities.
- Type: Any modality that does not impose excessive orthopedic stress; walking is the most common type of activity. Aquatic exercise and stationary cycle exercise may be advantageous for those with limited tolerance for weight bearing activity.

### Resistance exercise for older adults:

- Frequency: At least 2 d/wk.
- Intensity: Between moderate- (5-6) and vigorous- (7-8) intensity on a scale of 0 to 10.
- Type: Progressive weight training program or weight bearing calisthenics (8-10 exercises involving the major muscle groups of 8-12 repetitions each), stair climbing, and other strengthening activities that use the major muscle groups.

### Flexibility exercise for older adults:

- Frequency: At least 2 d/wk.
- Intensity: Moderate (5-6) intensity on a scale of 0 to 10.
- Type: Any activities that maintain or increase flexibility using sustained stretches for each major muscle group and static rather than ballistic movements.

Balance exercise for frequent fallers or individuals with mobility problems: ACSM/AHA Guidelines recommend balance exercise for individuals who are frequent fallers or for individuals with mobility problems because of a lack of adequate research evidence.

Exercise Prescription Guidelines recommend using activities that include the following: 1) progressively difficult postures that gradually reduce the base of support (e.g., two-legged stand, semi-tandem stand, tandem stand, one-legged stand), 2) dynamic movements that perturb the center of gravity (e.g., tandem walk, circle turns), 3) stressing postural muscle groups (e.g., heel stands, toe stands), or 4) reducing sensory input (e.g., standing with eyes closed).

The ACSM/AHA Guidelines recommend the following special considerations when prescribing exercise and physical activity for older adults. The intensity and duration of physical activity should be low at the outset for older adults who are highly deconditioned, functionally limited, or have chronic conditions that affect their ability to perform physical tasks. The progression of activities should be individual and tailored to tolerance and preference; a conservative approach may be necessary for the most deconditioned and physically limited older adults. Muscle strengthening activities and/or balance training may need to precede aerobic training activities among very frail individuals. Older adults should exceed the recommended minimum amounts of physical activity if they desire to improve their fitness. If chronic conditions preclude activity at the recommended minimum amount, older adults should perform physical activities as tolerated so as to avoid being sedentary.

The delivery of nutritional compositions to animals, such as human patients, that cannot orally ingest food or other forms of nutrition is often of critical importance. For example, feeding tubes that deposit food directly into the gastrointestinal tract at a point below the mouth are often used to sustain life while a patient is unable, or refuses, to take food orally. Feeding tubes and other artificial delivery systems and routes can be used temporarily during the treatment of acute conditions. For chronic conditions, such systems and routes can be used as part of a treatment regimen that lasts for the remainder of a patient's life. No matter the duration of use, these devices often provide the only means for feeding the patient.

In a preferred embodiment, the nutritional intervention will contain at least one of: protein, carbohydrate, fiber, fat, fatty acid, vitamin, mineral, sugar, carbohydrate, and flavor agent.

In one embodiment, the nutritional intervention will contain a liquid thickener. A liquid thickener is any additive that will increase the viscosity of a nutritional intervention to aid those patients that can benefit from a thickened liquid, such as those patient that have dysphagia. Many liquid thickeners are known in the art, but some examples include Xanthan gum, guar gum, locust bean gum, and a tare gum, tamarind gum, tragacanth gum, karaya gum, konjak mannan, CMC sodium, sodium alginate, pectin, azotobacter BINERANJIGAMU, carrageenan, an agar, gellant gum, a furcellaran, gelatin, curdlan, cassia gum, a psyllium seed gum, CMC carageenan, beta-glucan, modified starch, and starch.

One preferred embodiment of the nutritional intervention includes a formulation of a nutritional product with high protein content of (35-60% of total calories) provided as whey protein. The form of whey protein can be whey protein micelles or whey protein concentrate or isolate. The composition will also provide 10-30% of total calories as carbohydrate and 20-40% of total calories as fat. The protein component is inherently high in branched chain amino acids (leucine, valine, isoleucine) which have been shown to stimulate muscle synthesis. An advantage of providing these amino acids in this form is it not only avoids the adverse sensory impact of added amino acids, but also creates a favorable physiological response due to the high protein composition.

Another preferred embodiment of the nutritional intervention includes a formulation of a nutritional product with high protein content of (35-60% of total calories of the composition) provided as whey protein micelles. The composition will also provide 10-30% of total calories as carbohydrate and 20-40% of total calories as fat.

In a preferred embodiment of the invention, the methods of attenuating the loss of functional status comprising:
a) a nutritional intervention; and
b) an exercise regimen.

In a preferred embodiment of the invention, the methods of attenuating the loss of functional status comprising:
a) a nutritional intervention that helps prevent the loss of muscle mass; and
b) an exercise regimen.

In a preferred embodiment of the invention, the methods of attenuating the loss of functional status comprising:
a) a nutritional intervention that helps prevent the loss of muscle mass, said nutritional intervention comprising: Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof; and
b) an exercise regimen.

In a preferred embodiment of the invention, the methods of attenuating the loss of functional status comprising:
a) a nutritional intervention that helps prevent sarcopenia; and
b) an exercise regimen.

In a preferred embodiment of the invention, the methods of attenuating the loss of functional status comprising:
a) a nutritional intervention that helps prevent sarcopenia, said nutritional intervention comprising: vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof; and
b) an exercise regimen.

In a preferred embodiment of the invention, the methods of attenuating the loss of functional status comprising:
a) a nutritional intervention that helps prevent the loss of muscle function; and
b) an exercise regimen.

In a preferred embodiment of the invention, the methods of attenuating the loss of functional status comprising:
a) a nutritional intervention that helps prevent the loss of muscle function, said nutritional intervention comprising carotenoids; Vitamin D including Vitamin D3, 1,25 Dihydroxy Vitamin D, 25-Hydroxy Vitamin D; whey protein or any combination thereof; and
b) an exercise regimen.

In a preferred embodiment of the invention, the methods of attenuating the loss of functional status comprising:
a) a nutritional intervention that helps reduce inflammation; and
b) an exercise regimen.

In a preferred embodiment of the invention, the methods of attenuating the loss of functional status comprising:
a) a nutritional intervention that helps reduce inflammation, said nutritional intervention comprising Vitamin C; omega-3 fatty acids; Lactowolfberry or combinations thereof; and
b) an exercise regimen.

In a preferred embodiment of the invention, the methods of attenuating the loss of functional status comprising:
a) a nutritional intervention that helps reduce subclinical inflammation; and
b) an exercise regimen.

In a preferred embodiment of the invention, the methods of attenuating the loss of functional status comprising:
a) a nutritional intervention that helps reduce subclinical inflammation, said nutritional intervention comprising Vitamin C; omega-3 fatty acids; Lactowolfberry or combinations thereof; and
b) an exercise regimen.

In a preferred embodiment of the invention, the methods of attenuating the loss of functional status comprising:
a) a nutritional intervention that helps reduce calcium loss; and
b) an exercise regimen.

In a preferred embodiment of the invention, the methods of attenuating the loss of functional status comprising:
a) a nutritional intervention that helps reduce calcium loss, said nutritional intervention comprising Vitamin D including Vitamin D3, 1,25 Dihydroxy Vitamin D, 25-Hydroxy Vitamin D; calcium; whey protein and protein or any combination thereof; and
b) an exercise regimen.

In a preferred embodiment of the invention, the methods of attenuating the loss of functional status comprising:
a) a nutritional intervention that helps reduce oxidative stress; and
b) an exercise regimen.

In a preferred embodiment of the invention, the methods of attenuating the loss of functional status comprising:
a) a nutritional intervention that helps reduce oxidative stress, said nutritional intervention comprising antioxidants; and
b) an exercise regimen.

In a preferred embodiment of the invention, the methods of attenuating the loss of functional status comprising:
a) a nutritional intervention that helps reduce oxidative stress, said nutritional intervention comprising superoxide dismutases; glutathione; glutamine; Cysteine; cystine; glycine and whey protein or any combination thereof; and
b) an exercise regimen.

In a preferred embodiment of the invention, the methods of attenuating the loss of functional status comprising:
a) a nutritional intervention that helps reduce free radicals; and
b) an exercise regimen.

In a preferred embodiment of the invention, the methods of attenuating the loss of functional status comprising:
a) a nutritional intervention that helps reduce free radicals, said nutritional intervention comprising antioxidants; and
b) an exercise regimen.

In a preferred embodiment of the invention, the methods of attenuating the loss of functional status comprising:
a) a nutritional intervention that helps reduce free radicals, said nutritional intervention comprising Superoxide dismutases; glutathione; glutamine; Cysteine; cystine; glycine and whey protein or any combination thereof; and
b) an exercise regimen.

In a preferred embodiment of the invention, the methods of attenuating the loss of functional status comprising:
a) a nutritional intervention that helps reduce lactic acid; and
b) an exercise regimen.

In a preferred embodiment of the invention, the methods of attenuating the loss of functional status comprising:
a) a nutritional intervention that helps reduce lactic acid, said nutritional intervention comprising beta-alanine; and
b) an exercise regimen.

In a preferred embodiment of the invention, the methods of attenuating the loss of functional status comprising:
a) a nutritional intervention that helps promote protein synthesis; and
b) an exercise regimen.

In a preferred embodiment of the invention, the methods of attenuating the loss of functional status comprising:
a) a nutritional intervention that helps promote protein synthesis, said nutritional intervention comprising vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof; and
b) an exercise regimen.

In a preferred embodiment of the invention, the methods of attenuating the loss of functional status comprising:
a) a nutritional intervention that helps reduce protein catabolism; and
b) an exercise regimen.

In a preferred embodiment of the invention, the methods of attenuating the loss of functional status comprising:
a) a nutritional intervention that helps reduce protein catabolism, said nutritional intervention comprising vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof; and
b) an exercise regimen.

In a preferred embodiment of the invention, the methods of attenuating the loss of functional status comprising:
a) a nutritional intervention, wherein said nutritional intervention comprises at least two of:
   1) a nutritional intervention that helps prevent the loss of muscle mass;
   2) a nutritional intervention that helps prevent sarcopenia;
   3) a nutritional intervention that helps prevent the loss of muscle function;
   4) a nutritional intervention that helps reduce inflammation;
   5) a nutritional intervention that helps reduce subclinical inflammation;
   6) a nutritional intervention that helps reduce calcium loss;
   7) a nutritional intervention that helps reduce oxidative stress;
   8) a nutritional intervention that helps reduce free radicals;
   9) a nutritional intervention that helps reduce lactic acid;
   10) a nutritional intervention that helps promote protein synthesis;
   11) a nutritional intervention that helps reduce protein catabolism; and
b) an exercise regimen.

In a preferred embodiment of the invention, the methods of attenuating the loss of functional status comprising:
a) a nutritional intervention, wherein said nutritional intervention comprises at least two of:
   1) a nutritional intervention that helps prevent the loss of muscle mass, said nutritional intervention comprising: Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof;
   2) a nutritional intervention that helps prevent sarcopenia, said nutritional intervention comprising: vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof;
   3) a nutritional intervention that helps prevent the loss of muscle function, said nutritional intervention comprising carotenoids; Vitamin D including Vitamin D3, 1,25 Dihydroxy Vitamin D, 25-Hydroxy Vitamin D; whey protein or any combination thereof;
   4) a nutritional intervention that helps reduce inflammation, said nutritional intervention comprising Vitamin C; omega-3 fatty acids; Lactowolfberry or combinations thereof;
   5) a nutritional intervention that helps reduce subclinical inflammation, said nutritional intervention comprising Vitamin C; omega-3 fatty acids; Lactowolfberry or combinations thereof;
   6) a nutritional intervention that helps reduce calcium loss, said nutritional intervention comprising Vitamin D including Vitamin D3, 1,25 Dihydroxy Vitamin D, 25-Hydroxy Vitamin D; calcium; whey protein and protein or any combination thereof;
   7) a nutritional intervention that helps reduce oxidative stress, said nutritional intervention comprising superoxide dismutases; glutathione; glutamine; Cysteine; cystine; glycine and whey protein or any combination thereof;
   8) a nutritional intervention that helps reduce free radicals, said nutritional intervention comprising antioxidants; Superoxide dismutases; glutathione; glutamine; Cysteine; cystine; glycine and whey protein or any combination thereof;
   9) a nutritional intervention that helps reduce lactic acid, said nutritional intervention comprising beta-alanine;
   10) a nutritional intervention that helps promote protein synthesis, said nutritional intervention comprising vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof;
   11) a nutritional intervention that helps reduce protein catabolism, said nutritional intervention comprising vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof; and
b) an exercise regimen.

A benefit of at least one preferred embodiment of the invention, is the risk of morbidity is reduced.

In a preferred embodiment of the invention, the nutritional intervention is a complete nutrition.

In a preferred embodiment of the invention, the nutritional intervention is an incomplete nutrition.

In a preferred embodiment of the invention, the nutritional intervention is for short term administration.

In a preferred embodiment of the invention, the nutritional intervention is for long term administration.

In a preferred embodiment of the invention, the nutritional intervention is a tube feed.

In a preferred embodiment of the invention, the nutritional intervention is a gel.

In a preferred embodiment of the invention, the nutritional intervention is a concentrated liquid.

In a preferred embodiment of the invention, the nutritional intervention is a nutrient dense liquid.

In a preferred embodiment of the invention, the nutritional intervention further comprises a liquid thickener.

In a preferred embodiment of the invention, the nutritional intervention further comprises a liquid thickener.

In a preferred embodiment of the invention, the nutritional intervention will be administered in one dose from about one hour prior to the exercise regimen to about one hour after the exercise regimen.

In a preferred embodiment of the invention, the nutritional intervention will be administered in more than one dose from about one hour prior to the exercise regimen to about one hour after the exercise regimen.

In a preferred embodiment of the invention, the nutritional intervention will be administered in one dose from about 45 minutes prior to the exercise regimen to about 45 minutes after the exercise regimen.

In a preferred embodiment of the invention, the nutritional intervention will be administered in more than one dose from about 45 minutes prior to the exercise regimen to about 45 minutes after the exercise regimen.

In a preferred embodiment of the invention, the nutritional intervention will be administered in one dose from about 30 minutes prior to the exercise regimen to about 30 minutes after the exercise regimen.

In a preferred embodiment of the invention, the nutritional intervention will be administered in more than one dose from about 30 minutes prior to the exercise regimen to about 30 minutes after the exercise regimen.

In a preferred embodiment of the invention, the nutritional intervention will be administered in one dose from about 15 minutes prior to the exercise regimen to about 45 minutes after the exercise regimen.

In a preferred embodiment of the invention, the nutritional intervention will be administered in more than one dose from about 15 minutes prior to the exercise regimen to about 45 minutes after the exercise regimen.

In a preferred embodiment of the invention, the nutritional intervention will be administered in one dose from about 15 minutes prior to the exercise regimen to about 30 minutes after the exercise regimen.

In a preferred embodiment of the invention, the nutritional intervention will be administered in more than one dose from about 15 minutes prior to the exercise regimen to about 30 minutes after the exercise regimen.

In a preferred embodiment of the invention, the nutritional intervention will further provide hydration.

In a preferred embodiment of the invention, the nutritional intervention will prevent dehydration.

In a preferred embodiment of the invention, the nutritional intervention will prevent or diminish increased age-related arterial stiffening due to an exercise regimen.

In a preferred embodiment of the invention, the exercise regimen comprises endurance training.

In a preferred embodiment of the invention, the exercise regimen comprises resistance training.

In a preferred embodiment of the invention, the exercise regimen comprises endurance training and resistance training.

In a preferred embodiment of the invention, the exercise regimen is sufficient for release of myoD protein.

In a preferred embodiment of the invention, the exercise regimen is sufficient to activate satellite cells for muscles to repair and rebuild.

In a preferred embodiment of the invention, the exercise regimen is sufficient to stimulate the generation of more mitochondria within the myocytes.

In a preferred embodiment of the invention, the exercise regimen is sufficient to increase strength and power-generating capacity of the patient

In a preferred embodiment of the invention, said method is for use with an animal that can benefit from said method.

In a preferred embodiment of the invention, said method is for use in a mammal that can benefit from said method.

In a preferred embodiment of the invention, said method is for use in a human that can benefit from said method.

In a preferred embodiment of the invention, said method is for use in an elderly human that can benefit from said method.

In a preferred embodiment of the invention, the nutritional composition to attenuate the loss of functional status and help prevent the loss of muscle mass, said nutritional composition comprising: Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof.

In a preferred embodiment of the invention, the nutritional composition to attenuate the loss of functional status and help prevent sarcopenia, said nutritional composition comprising: vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof.

In a preferred embodiment of the invention, the nutritional composition to attenuate the loss of functional status and help prevent the loss of muscle function, said nutritional composition comprising carotenoids; Vitamin D including Vitamin D3, 1,25 Dihydroxy Vitamin D, 25-Hydroxy Vitamin D; whey protein or any combination thereof.

In a preferred embodiment of the invention, the nutritional composition to attenuate the loss of functional status and help reduce inflammation, said nutritional composition comprising Vitamin C; omega-3 fatty acids; Lactowolfberry or combinations thereof.

In a preferred embodiment of the invention, the nutritional composition to attenuate the loss of functional status and help reduce subclinical inflammation, said nutritional composition comprising Vitamin C; omega-3 fatty acids; Lactowolfberry or combinations thereof.

In a preferred embodiment of the invention, the nutritional composition to attenuate the loss of functional status and help reduce calcium loss, said nutritional composition comprising Vitamin D including Vitamin D3, 1,25 Dihydroxy Vitamin D, 25-Hydroxy Vitamin D; calcium; whey protein and protein or any combination thereof.

In a preferred embodiment of the invention, the nutritional composition to attenuate the loss of functional status and help reduce oxidative stress, said nutritional composition comprising superoxide dismutases; glutathione; glutamine; cysteine; cystine; glycine and whey protein or any combination thereof.

In a preferred embodiment of the invention, the nutritional composition to attenuate the loss of functional status and help reduce free radicals, said nutritional composition comprising antioxidants; Superoxide dismutases; glutathione; glutamine; cysteine; cystine; glycine and whey protein or any combination thereof.

In a preferred embodiment of the invention, the nutritional composition to attenuate the loss of functional status and help reduce lactic acid, said nutritional composition comprising beta-alanine.

In a preferred embodiment of the invention, the nutritional composition to attenuate the loss of functional status and help promote protein synthesis, said nutritional composition comprising vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof

In a preferred embodiment of the invention, the nutritional composition to attenuate the loss of functional status and help reduce protein catabolism, said nutritional composition comprising vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof

In a preferred embodiment of the invention, the nutritional composition to attenuate the loss of functional status, wherein said nutritional composition comprises at least two of:
1) a nutritional composition that helps prevent the loss of muscle mass;
2) a nutritional composition that helps prevent sarcopenia;
3) a nutritional composition that helps prevent the loss of muscle function;
4) a nutritional composition that helps reduce inflammation;
5) a nutritional composition that helps reduce subclinical inflammation;
6) a nutritional composition that helps reduce calcium loss;
7) a nutritional composition that helps reduce oxidative stress;
8) a nutritional composition that helps reduce free radicals;
9) a nutritional composition that helps reduce lactic acid;
10) a nutritional composition that helps promote protein synthesis; and
11) a nutritional composition that helps reduce protein catabolism.

In a preferred embodiment of the invention, the nutritional composition to attenuate the loss of functional status, wherein said nutritional composition comprises at least two of:
1) a nutritional composition that helps prevent the loss of muscle mass, said nutritional composition comprising: Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof;
2) a nutritional composition that helps prevent sarcopenia, said nutritional composition comprising: vitamins; minerals; zinc; magnesium; folate; vitamins C; Vitamin B 12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof;
3) a nutritional composition that helps prevent the loss of muscle function, said nutritional composition comprising carotenoids; Vitamin D including Vitamin D3, 1,25 Dihydroxy Vitamin D, 25-Hydroxy Vitamin D; whey protein or any combination thereof;
4) a nutritional composition that helps reduce inflammation, said nutritional composition comprising Vitamin C; omega-3 fatty acids; Lactowolfberry or combinations thereof;
5) a nutritional composition that helps reduce subclinical inflammation, said nutritional composition comprising Vitamin C; omega-3 fatty acids; Lactowolfberry or combinations thereof;
6) a nutritional composition that helps reduce calcium loss, said nutritional composition comprising Vitamin D including Vitamin D3, 1,25 Dihydroxy Vitamin D, 25-Hydroxy Vitamin D; calcium; whey protein and protein or any combination thereof;
7) a nutritional composition that helps reduce oxidative stress, said nutritional composition comprising superoxide dismutases; glutathione; glutamine; Cysteine; cystine; glycine and whey protein or any combination thereof;
8) a nutritional composition that helps reduce free radicals, said nutritional composition comprising antioxidants; Superoxide dismutases; glutathione; glutamine; Cysteine; cystine; glycine and whey protein or any combination thereof;
9) a nutritional composition that helps reduce lactic acid, said nutritional composition comprising beta-alanine;
10) a nutritional composition that helps promote protein synthesis, said nutritional composition comprising vitamins; minerals; zinc; magnesium; folate; vitamins C; Vitamin B 12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof; and
11) a nutritional composition that helps reduce protein catabolism, said nutritional composition comprising vitamins; minerals; zinc; magnesium; folate; vitamins C; Vitamin B12, Whey protein; creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof

In a preferred embodiment of the invention, the nutritional composition further comprises at least one of: protein, carbohydrate, fiber, fat, fatty acid, vitamin, mineral, sugar, carbohydrate, and flavor agent.

In a preferred embodiment the nutritional intervention is a 125 ml serving that is: Energy 200 kcal; Protein 20 g (100% Whey Protein Micelles); Carbohydrate 7.2 g; Fat 7.2 g; Vitamin D3 13µg; with added: Sodium, Chloride, Potassium, Calcium, Phosphorus, Magnesium, Iron, Zinc, Copper, Iodine, Selenium, Manganese, Chromium, Molybdenum, Fluoride, Vitamin A, Vitamin E, Vitamin K, Vitamin C, Vitamin B1, Vitamin B2, Vitamin B6, Niacin, Folic acid, Vitamin B12, Pantothenic acid, Biotin, Choline, Taurine, and Carnitine.

In a preferred embodiment of the invention, the nutritional composition is a complete nutrition.

In a preferred embodiment of the invention, the nutritional composition is an incomplete nutrition.

In a preferred embodiment of the invention, the nutritional composition is for short term administration.

In a preferred embodiment of the invention, the nutritional composition is for long term administration.

In a preferred embodiment of the invention, the nutritional composition is a tube feed.

In a preferred embodiment of the invention, the nutritional composition is a gel.

In a preferred embodiment of the invention, the nutritional composition further comprises a liquid thickener.

In a preferred embodiment of the invention, the method will also include at least one behavioral component such as: interaction with other people, interaction with animals (such as a dog or cats), psychological counseling, stress management, grief management, depression management, and dementia management.

In a preferred embodiment of the invention, the method will also include at least one cognitive component such as: reading, mind stimulating activities, problem solving activities, writing activities, puzzles, interactive games, and video games.

The foregoing description of various aspects of the invention has been presented for purposes of illustration and description. It is not intended to be exhaustive or to limit the invention to the precise form disclosed, and obviously, many modifications and variations are possible. Such modifications and variations that may be apparent to a person skilled in the art are intended to be included within the scope of the invention as defined by the accompanying claims.

## Claims

1. A method of attenuating the loss of functional status comprising:
a) a nutritional intervention that helps prevent the loss of muscle mass, said nutritional intervention comprising: Whey protein and at least one of: creatine; antioxidants; bioactives; lycopene; pycnogenol; quercetin; genistein; soy; epigallocatechin; green tea; lipoic acid; Alpha-Lipoic Acid; X-tocopherol; dihydroascorbic acid; Vitamin C; Vitamin D; ubiquinone; Coenzyme Q10; Leucine; Leucine metabolites; alpha-hydroxyisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; or nucleotides; and
b) an exercise regimen.

2. The method of claim 1, wherein said whey protein comprises whey protein isolate, whey protein concentrate, whey protein hydrolysates, whey protein micelles or combinations thereof

3. The method as claimed in Claim 1, wherein the whey protein is whey protein micelles.

4. The method of claim 1, wherein said nutritional intervention further comprises at least one of carotenoids; Vitamin D; or Vitamin D3.

5. The method of claim 1, wherein said nutritional intervention comprises whey protein micelles and Vitamin D3.

6. The method as claimed in Claim 1, wherein the nutritional intervention helps prevent sarcopenia.

7. The method as claimed in Claim 1, wherein the nutritional intervention helps prevent the loss of muscle function.

8. The method as claimed in Claim 1, wherein the nutritional intervention helps reduce inflammation, said nutritional intervention further comprising Vitamin C; omega-3 fatty acids; or Lactowolfberry or combinations thereof.

9. The method as claimed in Claim 1, wherein the nutritional intervention helps reduce oxidative stress, said nutritional intervention further comprising: superoxide dismutases; glutathione; glutamine; Cysteine; cystine; or glycine or any combination thereof.

10. The method as claimed in Claim 1, wherein the nutritional intervention helps promote protein synthesis, said nutritional intervention further comprising vitamins; minerals; zinc; magnesium; folate; vitamins C; Vitamin B 12, creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; or nucleotides or any combination thereof.

11. The method as claimed in Claim 1, wherein the nutritional intervention helps reduce protein catabolism, said nutritional intervention further comprising vitamins; minerals; zinc; magnesium; folate; vitamins C; Vitamin B 12, creatine; antioxidants including bioactives such as lycopene, pycnogenol; quercetin; genistein; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; or nucleotides or any combination thereof.

12. The method as claimed in Claim 1, wherein the nutritional intervention is a complete nutrition.

13. The method as claimed in Claim 1, wherein the nutritional intervention further comprises a liquid thickener.

14. The method as claimed in Claim 1, wherein the nutritional intervention will be administered in one or more doses from about one hour prior to the exercise regimen to about one hour after the exercise regimen.

15. The method as claimed in Claim 1, wherein the exercise regimen comprises endurance training or resistance training or endurance training and resistance training.

16. The method as claimed in Claim 1, further comprising a behavioral component and/or a cognitive component.

17. A nutritional composition to attenuate the loss of functional status and help prevent the loss of muscle mass, said nutritional composition comprising:
i) Whey protein comprising whey protein isolate, whey protein concentrate, whey protein hydrolysates, whey protein micelles or combinations thereof, and
ii) at least one of: creatine; antioxidants; bioactives; lycopene, pycnogenol; quercetin; genistein; soy; epigallocatechin; green tea; lipoic acid; Alpha-Lipoic Acid; X-tocopherol; dihydroascorbic acid; Vitamin C; Vitamin D; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides or any combination thereof.

18. The nutritional composition as claimed in Claim 17, wherein further comprises Vitamin D3.

19. The nutritional composition as claimed in Claim 17, comprising whey protein micelles and Vitamin D3.

20. The nutritional composition as claimed in Claim 17, further comprising vitamins; minerals; zinc; magnesium; folate; vitamins C; Vitamin B12, Whey protein; creatine; antioxidants including bioactives such as lycopene, pycnogenol; quercetin; genistein; lactowolberry; soy; epigallocatechin; green tea; lipoic acid; Alpha-Lipoic Acid; X-tocopherol; dihydroascorbic acid; Vitamin C; ubiquinone; Coenzyme Q10; Leucine; Leucine metabolites; alpha-hydroxuisocaproate; beta-hydroxy beta-methylbutyrate; keto-isopcaproate; branched chain amino acid(s) (BCAA), a BCAA precursor; a BCAA metabolite; a BCAA-rich protein; a protein manipulated to enrich the BCAA content; nucleotides ; omega-3 fatty acids or any combination thereof.

21. The nutritional composition as claimed in Claim 17, wherein the nutritional composition is a complete nutrition.

22. The nutritional composition as claimed in Claim 17, wherein the nutritional composition further comprises a liquid thickener.
